# EUROPÄISCHE PATENTSCHRIFT

(11) **EP 2 153 869 B1**
(45) Veröffentlichungstag und Bekanntmachung des Hinweises auf die Patenterteilung: **29.03.2017**
(21) Anmeldenummer: 09010291.4
(22) Anmeldetag: 10.08.2009
(51) Int. Cl.: A61N 1/40, A61N 2/02

(54) **Befeldungsgerät zur Befeldung von Körperteilen von Lebewesen mit elektromagnetischen Wellen**
Irradiation device for irradiating body parts of living organisms with electromagnetic waves
Appareil de rayonnement destiné au rayonnement de parties du corps d'humains avec des ondes électromagnétiques

(30) Priorität: 16.08.2008 DE 102008037979
(43) Veröffentlichungstag der Anmeldung: 17.02.2010
(73) Patentinhaber: Pries, Wolfgang, 86825 Bad Wörishofen (DE)
(72) Erfinder: Pries, Wolfgang, 86825 Bad Wörishofen (DE)
(74) Vertreter: Liebl, Thomas

(56) Entgegenhaltungen:
- EP-A1- 0 500 983
- EP-B1- 0 136 530
- DE-A1- 10 100 385
- DE-A1-102005 014 023
- US-A1- 2004 077 921

## Beschreibung

Die Erfindung betrifft ein Befeldungsgerät zur Befeldung von Körperteilen von Lebewesen mit elektromagnetischen Wellen nach dem Oberbegriff des Patentanspruchs 1.

Ein bekanntes, gattungsgemäßes Befeldungsgerät (DE 0136 530 B1) enthält eine Elektronikeinheit mit Energieversorgung bestehend aus einer Hochfrequenzoszillatorstufe zur Erzeugung eines Hochfrequenzwellenzuges mit einer Frequenz im Bereich von unter anderem 130 MHz bis 170 MHz sowie einer Niederfrequenzoszillatorstufe, die eine Frequenz als Modulationsfrequenz erzeugt. Der Ausgang der Hochfrequenzoszillatorstufe und der Ausgang der Niederfrequenzoszillatorstufe werden einer Modulationseinheit zugeführt, wodurch ein modulierter Wellenzug gebildet wird, der in einem nachgeordneten Endverstärker als Leistungsstufe verstärkt wird. An den Endverstärker ist eine Sendeantenne angeschlossen in deren Abstrahlbereich ein Körperteil zur Befeldung positionierbar ist.

Beim bekannten Befeldungsgerät ist zudem ein Taktgenerator vorgesehen, der den modulierten Wellenzug mit einer einstellbaren Taktfrequenz von 0,5 Hz bis 40 Hz jeweils unterbricht. Bei einem ähnlichen Befeldungsgerät ist eine gleichzeitige akustische Befeldung mit einer Schallwelle vorgesehen mit einer der niederfrequenten Modulationsfrequenz oder einem ganzzeiligen vielfachen davon entsprechenden Schallwellenfrequenz (DE 101 00 385 A1).

Weiter ist ein Befeldungsgerät bekannt bei dem die Sendeantenne aus wenigstens einer Befeldungskapsel besteht, die zumindest eine Magnetspule enthält, wobei die Befeldungskapsel am Körperteil eines Lebewesens anlegbar ist (DE 10 2005 014 023 A1). Zudem ist hier eine Feldstärke-Begrenzungseinrichtung für die Begrenzung der von der Magnetspule erzeugten Magnetspulen-Feldstärke vorgesehen, um unzulässig hohe unter ungünstigen Bedingungen von der Befeldungskapsel in die Umgebung möglicherweise abgestrahlte Hochfrequenz-Störpegel (HF-Störpegel) zu vermeiden.

Bei der in der US 2004/077921 A1 gezeigten Vorrichtung handelt es sich um ein Gerät, welches vorzugsweise mit 12 Volt Gleichspannung betrieben wird und lediglich variierende Magnetfelder mit sehr geringer Magnetfeldgröße entsprechend natürlicher Magnetfelder erzeugen soll. Die Variation kann in einem Bereich von 0 bis 45 Hz, vorzugsweise bei 9,6 Hz eingestellt sein. Zur Befeldung sind hier lediglich einfache offene Magnetspulen vorgesehen - wie diese am oder im Bereich eines Patienten angebracht werden können ist nicht näher gezeigt.

In der EP 0 500 983 A1 ist eine Bestrahlungsvorrichtung zur Behandlung von lebendem Gewebe mit elektromagnetischen Wellen beschrieben. Es ist dabei nur eine Antenne gezeigt in derem Abstrahlbereich der Patient oder das zu bestrahlende Gewebe liegen soll. Weiter soll hier die Niederfrequenz als Modulationsfrequenz über einen Frequenzdurchlauf nach einer frequenzspezifischen Patientenreaktion selbsttätig vom Gerät eingestellt werden. Ersichtlich erfordert dies ein teueres aufwändiges Gerät mit dafür geeigneten Sensoren und Regelkreisen.

Alle vorstehend genannten Befeldungsgeräte sind für einen breiten Einsatzbereich konzipiert mit einer Vielzahl von Einstellmöglichkeiten über große Frequenzbereiche der hochfrequenten Trägerfrequenz und der niederfrequenten Modulationsfrequenz in Verbindung mit Einstellmöglichkeiten für Befeldungsleistungen, Zeitvorgaben, Taktfrequenzen für den Taktgenerator sowie Lautstärken und Frequenzen für akustische Beeinflussungen. Damit wird ein breites Therapiespektrum mit einer Vielzahl von Abstimmungen, Anpassungen und Optimierungen an eine entsprechende Vielzahl von Beschwerden von Patienten ermöglicht. Es ist daher erforderlich, dass aus dieser Vielzahl von Einstellmöglichkeiten definierte, auf den konkreten Behandlungsfall abgestellte Einstellungsvorgaben von einem Therapeuten vorgegeben werden und entweder von diesem oder einer mit dem Befeldungsgerät vertrauten Bedienperson eingestellt werden. Dadurch ist auch der ausschließliche Einsatz eines solchen Befeldungsgerätes in einer Arztpraxis oder einer anderen Therapieeinrichtung vorgegeben, wo auf die Bedienung des Gerätes spezialisierte Personen zur Verfügung stehen. Durch die vielen Befeldungs- und Einstellmöglichkeiten ist zudem ein solches Gerät kompliziert aufgebaut und entsprechend teuer, sodass sich auch aus diesem Grund nur die Anschaffung für eine Arztpraxis oder ein Therapieinstitut rechtfertigt, wo das Befeldungsgerät von einer Mehrzahl von Patienten im Wechsel und in Verbindung mit einer assistierenden Person benutzt werden kann. Durch die zentrale Aufstellung eines solchen Gerätes ergibt sich zwangsläufig, dass sich Patienten meist in regelmäßigen Abständen zur einer vorgegebenen Anzahl von Befeldungen am Ort des Befeldungsgeräts einfinden müssen. Dies ist beispielsweise bei arthrose- oder demenzkranken Patienten schwierig, beschwerlich und aufwendig und meist nur mit Hilfe von Begleitpersonen durchführbar. Bei zweckmäßigen Befeldungsdauern von bis zu 1 Stunde kann mit einem Befeldungsgerät nur eine kleine Anzahl von Patienten pro Tag behandelt werden.

Wegen der zunehmenden Überalterung der Gesellschaft nehmen altersbedingte Beschwerden und Krankheiten, wie beispielsweise Demenz und Arthrosen verstärkt zu. Eine positive Beeinflussung wäre unter Umständen mit den bekannten Befeldungseinrichtungen möglich, jedoch stehen die vorstehend erläuterten einschränkenden Umstände einem flächendeckenden Einsatz bei einer großen Patientenanzahl, insbesondere schon bei beginnenden Beschwerden, entgegen.

Aufgabe der Erfindung ist es daher, ein Befeldungsgerät zur Befeldung von Körperteilen von Lebewesen mit elektromagnetischen Wellen zur Verfügung zu stellen, das kostengünstig herstellbar ist und zudem direkt von einem Patienten einfach zu handhaben und zu bedienen ist, ohne dass speziell geschulte Personen erforderlich sind

Diese Aufgabe wird mit den Merkmalen des Anspruchs 1 gelöst.

Gemäß Anspruch 1 ist die Sendeantenne aus zwei am Endverstärker angeschlossenen Befeldungskapseln gebildet, die jeweils zumindest eine Magnetspule enthalten und die an einem Körperteil anlegbar sind, wobei die zwei Befeldungskapseln mit ihren aktiven Seiten aufeinander zugerichtet sind und durch eine bügelartige oder kappenartige Halterung miteinander verbunden sind, wobei die Halterung am zu befeldenden Körperteil so aufsetzbar ist, dass die Befeldungskapseln gegenüberliegend am Körperteil anliegen.

Zudem ist die elektrische Leistung der Leistungsstufe auf maximal 2 mW begrenzt. Einerseits wird mit einer solchen Leistung bereits die gewünschte positive Beeinflussung erreicht und andererseits führt die Anwendung bei dieser geringen Leistung zu keinen unerwünschten Temperatureffekten gegebenenfalls mit Gewebeschädigungen.

Wesentlich ist eine vorgegebene unveränderbare geräteinterne Einstellung der Modulationsfrequenz auf 10,0 Hz. Es hat sich durch umfangreiche Versuche in Verbindung mit theoretischen Überlegungen gezeigt, dass die Modulationsfrequenz von 10,0 Hz zu hervorragenden Erfolgen bei einer Arthrosebehandlung führt. Alternativ (oder geräteintern oder extern umstellbar) ist eine Modulationsfrequenz von 10,2 Hz fest eingestellt. In umfangreichen Langzeitversuchen hat sich gezeigt, dass die Modulationsfrequenz von 10,2 Hz zu herausragenden Erfolgen bei einer nichtinvasiven transkraniellen Stimulation in Verbindung mit einer Verbesserung der Hirnleistung bei einer Befeldung des Kopfbereiches führt. Da bereits geringe Veränderungen dieser Modulationsfrequenzen die Therapieerfolge erheblich reduzieren oder unterbinden ist die erfindungsgemäße Festeinstellung der Modulationsfrequenz 10,0 Hz und/oder 10,2 Hz ein wesentlicher Aspekt der Erfindung.

Auch die Hochfrequenzoszillatorstufe ist auf einen festen und unveränderbaren Frequenzwert geräteintern eingestellt, wobei eine Festeinstellung in einem Bereich zwischen 130 MHz und 170 MHz als Trägerfrequenz gut geeignet ist. Es hat sich gezeigt, dass ein therapeutischer Effekt im Wesentlichen durch die genaue Modulationsfrequenzeinstellung hervorgerufen wird, die Hochfrequenzeinstellung dagegen weniger kritisch ist sofern der vorstehende Bereich von 130 MHz bis 170 MHz eingehalten wird.

Weiter erfindungsgemäß ist die Elektronikeinheit in einem Gerätegehäuse enthalten, an dem ein betätigbarer Ein-Aus-Schalter für die Elektronikeinheit zu einer einfachen Selbstanwendung und Selbstbedienung durch einen Patienten angebracht ist, wobei die Halterung zudem das Gerätegehäuse zur Aufnahme der Elektronikeinheit mit dem Ein-Aus-Schalter ausbildet.

In der einfachsten Version eines solchen Geräts mit festen Einstellungen für die Modulationsfrequenz, die Trägerfrequenz, die Befeldungsleistung und gegebenenfalls die Befeldungsdauer ist somit als einziges Bedienungselement ein Ein-Aus-Schalter am Gerätegehäuse erforderlich.

Durch den einfachen Aufbau sind solche Befeldungsgeräte in größeren Serien einfach und so kostengünstig herstellbar, dass der Kauf gegebenenfalls unterstützt durch Krankenkassen unmittelbar vom Patienten möglich wird. Damit sind dezentrale Anwendungen bei und durch eine Vielzahl von Patienten direkt am Ort des Patienten flächendeckend möglich, was durch einfachste Bedienung und damit einen Ausschluss von Fehlbedienungen begünstigt wird. Damit ist lediglich die Diagnose beim Arzt zu erstellen, worauf dieser gegebenenfalls das vorliegende Befeldungsgerät und die Anzahl und den zeitlichen Abstand der Anwendung bestimmt - die Anwendung führt dagegen der Patient selbst nach Erwerb des Befeldungsgerätes in seinem Privatbereich durch. Mit den Befeldungsgeräten nach dem Stand der Technik war dies nicht möglich. Lediglich zur Überprüfung eines Therapieerfolgs muss dann ein Patient wieder die Arztpraxis aufsuchen.

Gemäß Anspruch 2 ist eine fest eingestellte unveränderbare Hochfrequenz von 150 MHz als Trägerfrequenz sehr gut geeignet.

Hervorragende Erfolge in der Arthrose-Therapie werden, wie vorstehend erläutert, mit einer Modulationsfrequenz von 10,0 Hz erreicht und entsprechend hervorragende Erfolge mit einer nichtinvasiven transkraniellen Stimulation mit einer Modulationsfrequenz von 10,2 Hz. Es können daher zwei unterschiedliche Gerätetypen hergestellt werden, die jeweils auf eine dieser Modulationsfrequenzen fest eingestellt sind. In einer alternativen Ausführungsform nach Anspruch 3 wird ein Befeldungsgerät vorgeschlagen, das für beide Anwendungen geeignet ist, indem zwischen den Modulationsfrequenzen 10,0 Hz und 10,2 Hz umgeschaltet werden kann. Für eine fehlerfreie Anwendung des Gerätes wird den jeweiligen zwei Wählstellungen eine Aufschrift "Arthrose-Therapie" und "Transkranielle Stimulation" zugeordnet.

In einer Weiterbildung nach Anspruch 4 wird mit dem Ein-Aus-Schalter ein Zeitglied gestartet, sodass nach einer festeingestellten Befeldungszeit das Befeldungsgerät wieder automatisch abschaltet und/oder ein akustisches Signal abgibt. Es ist auch möglich über ein zusätzliches Zeitvorgabeelement die Befeldungszeit vorwählbar einzustellen.

Für die Anwendung "Arthrose-Therapie" ist eine festeingestellte Befeldungszeit von 45 Minuten gut geeignet und für die Anwendung zu einer nichtinvasiven "Transkranielle Stimulation" entsprechend 60 Minuten. Bei einer Geräteausführung mit einer Umschaltmöglichkeit zwischen diesen beiden Anwendungen werden dann die vorstehenden Befeldungszeiten entsprechend den Anwendungen selbsttätig vorgewählt und zugeordnet.

Wesentlich an dem Befeldungsgerät ist eine Begrenzung der Befeldungsleistung auf einen Maximalwert von etwa 2 mW, da es sich hier um keine Hyperthermieanwendung handelt, sondern um eine Stimulation des befeldeten Gewebes und der Zellen. Um die Befeldungsleistung gegebenenfalls weiter reduzieren zu können kann nach Anspruch 6 zusätzlich ein Leistungsstellelement verwendet werden.

Aufgrund der geringen Befeldungsleistung ist insgesamt der Energieverbrauch des Befeldungsgeräts sehr gering, sodass sich dieses gemäß Anspruch 7 für einen netzunabhängigen Betrieb mit einem geräteseitigen Akku eignet. Dadurch ist die Handhabung weiter vereinfacht und bequem gestaltet. Um das Befeldungsgerät klein und gewichtsgünstig zu halten, wird weiter vorgeschlagen einen Transformator mit einem Steckanschluss zu einer Gebäudesteckdose geräteextern anzuordnen mit einer Kabelverbindung nur während der Ladezeit des Akkus.

Die Halterung wird vorteilhaft flexibel und gegebenenfalls in ihrer Weite einstellbar ausgeführt dergestalt, dass die Befeldungskapseln gegenüberliegend am befeldenden Körperteil, beispielsweise einem Kniegelenk anliegen und durch die Halterung in dieser Stellung leicht angepresst und fixiert werden.

Für eine Justierung und Anpassung an individuelle Gegebenheiten wird mit Anspruch 8 vorgeschlagen, dass die Halterung zwei Bügelteile aufweist, die über eine einstellbare Schiebeführung zur Abstandeinstellung der Befeldungskapseln miteinander verbunden sind. Das Anlegen der Befeldungskapseln ist damit sehr einfach durchführbar und die Befeldungskapseln werden während der Befeldungszeit ohne weiteres Zutun an der Anlagestelle fixiert und gehalten.

Für einen besonders kompakten, einfachen und kostengünstig herstellbaren Aufbau stellt die Halterung zwischen den Befeldungskapseln in einer weiteren Funktion zusätzlich das Gerätegehäuse zur Aufnahme der Elektronikeinheit und der Betätigungselemente dar, d.h. dass die Halterung zudem das Gerätegehäuse zur Aufnahme der Elektronikeinheit mit dem Ein-Aus-Schalter sowie mit einem Wählschalter für die Modulationsfrequenz von 10,0 Hz oder 10,2 Hz und/oder einem Zeitvorgabeelement als Drehknopf oder Taster oder Schalter und/oder einem Leistungs-Einstellelement und/oder einem zugeordneten Display und/oder einem Akku ausgebildet ist. Obwohl vorstehend die einzelnen Funktionselemente des Befeldungsgerätes, wie beispielsweise ein Hochfrequenzoszillator, ein Niederfrequenzoszillator, eine Modulationseinheit usw. als diskrete Bauelemente zur Verdeutlichung der Gesamtfunktion beschrieben und beansprucht sind, soll von der Beschreibung und vom Schutzumfang auch die Integration der Funktionselemente in integrierten Bauelementen mit umfasst sein. Durch Verwendung solcher integrierter und miniaturisierter Bauelemente und in Anbetracht der geringen erforderlichen Leistung ist die Aufnahme der gesamten Elektronik in einem Haltebügel zwischen den Befeldungskapseln problemlos möglich. Besonders vorteilhaft entfallen dann auch Kabel zwischen einem Gerätegehäuse und den Befeldungskapseln, da hier ein elektrischer Anschluss unmittelbar im Haltebügel möglich ist.

Das Befeldungsgerät kann nach Anspruch 9 mit einer an sich bekannten Feldstärke-Begrenzungsvorrichtung (DE 10 2005 014 023 A1) für eine Begrenzung der von den Befeldungskapseln in die Umgebung abgestrahlten Hochfrequenz-Störpegel (HF-Störpegel) ausgerüstet werden.

Es zeigen:
- Fig. 1: eine schematische Darstellung eines Befeldungsgerätes zur Befeldung von Körperteilen von Lebewesen mit einem Blockschaltbild der Elektronikeinheit;
- Fig. 2: einen modulierten Wellenzug und
- Fig. 3: ein kompakt aufgebautes Befeldungsgerät.

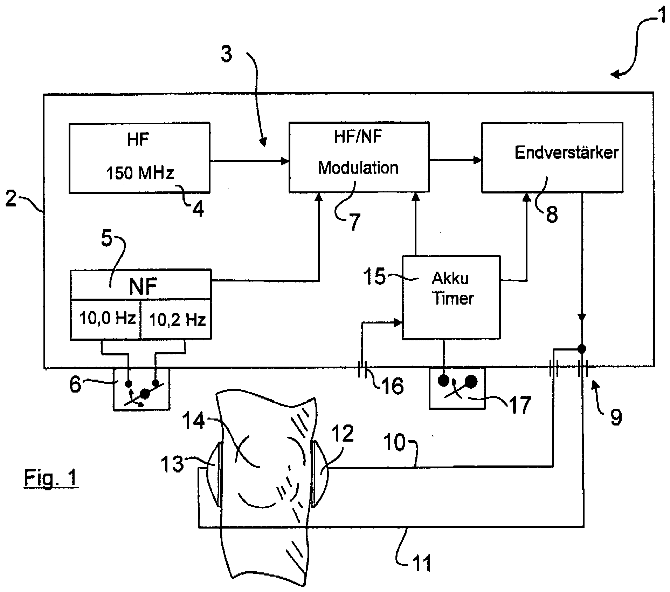

In Fig. 1 ist ein Befeldungsgerät 1 schematisch dargestellt mit einem Gerätegehäuse 2 in dem eine Elektronikeinheit 3 enthalten ist.

Die Elektronikeinheit 3 besteht aus einer Hochfrequenzoszillatorstufe 4, welche einen Hochfrequenzwellenzug mit einer Frequenz von 150 MHz erzeugt. Weiter umfasst die Elektronikeinheit 3 eine Niederfrequenzoszillatorstufe 5, die eine Frequenz von 10,0 Hz oder 10,2 Hz als Modulationsfrequenz erzeugt. Zwischen diesen beiden Frequenzen kann mit einem am Gerätegehäuse 2 angebrachten Wählschalter 6 umgeschaltet werden, wobei die Wählstellung für 10,0 Hz einer "Arthrose-Therapie" und die Wählstellung für 10,2 Hz einer "Transkraniellen Stimulation" zugeordnet ist.

Die Ausgänge der Hochfrequenzoszillatorstufe 4 und der Niederfrequenzoszillatorstufe 5 sind einer Modulationseinheit 7 zugeführt, in der ein entsprechend modulierter Wellenzug 22 gebildet wird, der in einem nachgeschalteten Endverstärker 8 verstärkt wird und für eine Befeldung an einem Doppelstecker 9 zur Verfügung steht.

An diesem Doppelstecker 9 sind über Kabel 10, 11 zwei Befeldungskapseln 12, 13 angeschlossen, welche hier jeweils seitlich gegenüberliegend an einem Kniegelenk 14 einer Person für eine Befeldung angelegt sind. Für einen guten Therapieerfolg soll anschließend an eine Befeldung mit der hier gezeigten seitlichen Anbringung der Befeldungskapseln 12, 13 eine weitere Befeldung mit um 90° versetzten Befeldungskapseln12, 13, das heißt in Längsrichtung durchgeführt werden.

Weiter umfasst die Elektronikeinheit eine Energieversorgungseinheit 15 zur Energieversorgung der Bauteile 4, 5, 7, 8, die hier konkret eine Akku enthält, welcher über einen Ladeanschluss 16 mit einem (nicht dargestellten) Ladekabel aufgeladen werden kann. Zudem umfasst die Energieversorgungseinheit 15 ein Zeitglied als Timer, hier mit einer fest vorgegebenen Befeldungszeit, der über einen am Gerätgehäuse 2 angebrachten Ein-Aus-Schalter 17 zum Start einer Befeldung eingeschaltet werden kann.

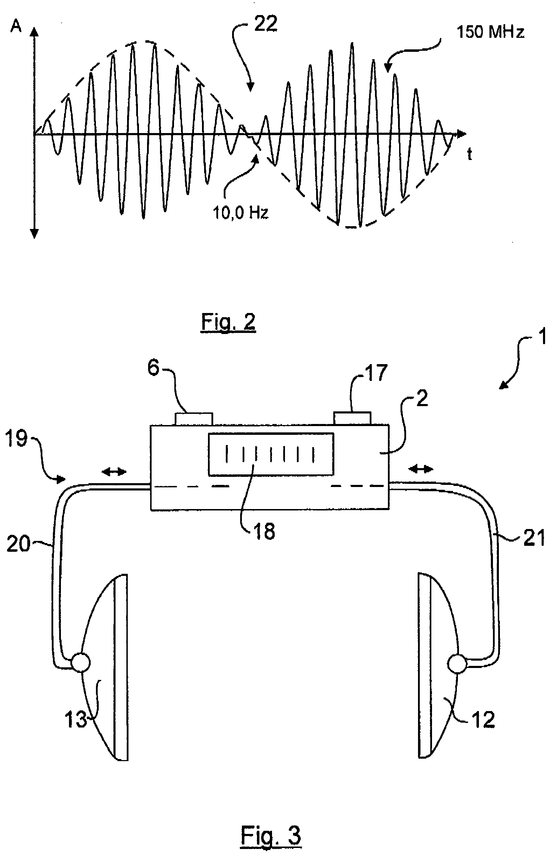

Fig. 2 zeigt lediglich schematisch den modulierten Wellenzug 22, der verstärkt am Ausgang des Endverstärkers 8 für eine Befeldung zur Verfügung steht, wobei das Verhältnis zwischen der Hochfrequenz und Niederfrequenz maßstäblich nicht exakt dargestellt ist.

In Fig. 3 ist eine bevorzugte konkrete Ausbildung des Befeldungsgerätes 1 dargestellt:
Das Gerätegehäuse 2 ist länglich, schmal und relativ klein sowie gewichtsgünstig ausgeführt. Zusätzlich zum Wählschalter 6 und dem Ein-Aus-Schalter 17 können je nach Ausführung noch weitere (nicht dargestellte) Bedienelemente sowie ein Display 18 vorgesehen sein.

Das Gerätegehäuse 2 ist hier Teil einer bügelförmigen Halterung 19, welche zudem zwei Bügelteile 20, 21 umfasst. Diese Bügelteile 20, 21 sind L-förmig abgewinkelt und tragen jeweils an einer Endseite aufeinander zugerichtet die Befeldungskapseln 12 und 13. Die anderen beiden L-Schenkel sind im Gerätegehäuse 2 für eine Abstandseinstellung der Befeldungskapseln 12, 13 gegeneinander verschiebbar und justierbar. Zudem sind die Bügelteile 20, 21 hohl und enthalten integrierte elektrische Anschlusskabel 10, 11. Für eine Befeldung eines Kniegelenks 14 werden die Bügelteile 20, 21 und damit die Befeldungskapseln 12, 13 vorerst auseinander gefahren und über das Kniegelenk 14 gesteckt. Anschließend werden die Bügelteile 20, 21 zusammengeschoben, bis die Befeldungskapseln 12, 13 am Kniegelenk 14 fest anliegen und dort fixiert sind. Da hier weder zusätzliche Maßnahmen zur Halterung der Befeldungskapseln noch anzuschließende Kabel erforderlich sind, ist die Handhabung und Bedienung ausgesprochen einfach.

## Patentansprüche

1. Befeldungsgerät zur Befeldung von Körperteilen von Lebewesen mit elektromagnetischen Wellen,
- mit einer Elektronikeinheit (3) mit Energieversorgung (15) bestehend aus
- einer Hochfrequenzoszillatorstufe (4) zur Erzeugung eines Hochfrequenzwellenzuges mit einer Frequenz im Bereich von 130 MHz bis 170 MHz,
- einer Niederfrequenzoszillatorstufe (5), die eine Frequenz als Modulationsfrequenz erzeugt.
- einer Modulationseinheit (7), der der Ausgang der Hochfrequenzoszillatorstufe (4) und der Ausgang der Niederfrequenzoszillatorstufe (5) zugeführt sind zur Bildung eines modulierten Wellenzuges, und
- einem nachgeordneten Endverstärker (8) als Leistungsstufe mit angeschlossener Sendeantenne, in deren Abstrahlbereich ein Körperteil (14) zur Befeldung positioniert ist,
**dadurch gekennzeichnet,**
**dass** die Sendeantenne aus zwei am Endverstärker (8) angeschlossenen Befeldungskapseln (12, 13) gebildet ist, die jeweils zumindest eine Magnetspule enthalten und die an einem Körperteil (14) anlegbar sind, wobei die Befeldungskapseln (12, 13) mit ihren aktiven Seiten aufeinander zugerichtet durch eine bügelartige oder kappenartige Halterung (19) miteinander verbunden sind, wobei die Halterung (19) am zu befeldenden Körperteil (14) so aufsetzbar ist, dass die Befeldungskapseln (12, 13) gegenüberliegend am Körperteil (14) anliegen,
**dass** die elektrische Leistung der Leistungsstufe (8) auf maximal 2 mW begrenzt ist,
**dass** die Modulationsfrequenz fest und unveränderbar auf 10,0 Hz und/oder 10,2 Hz eingestellt ist,
**dass** die Hochfrequenzoszillatorstufe (4) auf einen festen und unveränderbaren Frequenzwert im Bereich zwischen 130 MHz und 170 MHz eingestellt ist, und
**dass** die Elektronikeinheit (3) in einem Gerätegehäuse (2) enthalten ist, an dem ein betätigbarer Ein-Aus-Schalter (17) für die Elektronikeinheit (3) zu einer einfachen Selbstanwendung und Selbstbedienung durch einen Patienten angebracht ist, und
**dass** die Halterung (19) zudem das Gerätegehäuse (2) zur Aufnahme der Elektronikeinheit (3) mit dem Ein-Aus-Schalter (17) ausbildet.

2. Befeldungsgerät nach Anspruch 1, **dadurch gekennzeichnet, dass** der feste und unveränderbare Hochfrequenzwert auf 150 MHz eingestellt ist.

3. Befeldungsgerät nach Anspruch 1 oder 2, **dadurch gekennzeichnet, dass** die Modulationsfrequenz mit einem Wählschalter (6) am Gerätegehäuse (2) zwischen 10,0 Hz und 10,2 Hz umschaltbar ist und den beiden Wählstellungen die Markierungen "Arthrose-Therapie" und "Transkranielle Stimulation" zugeordnet sind.

4. Befeldungsgerät nach einem der Ansprüche 1 bis 3, **dadurch gekennzeichnet, dass** dem Ein-Aus-Schalter (17) eine Zeitvorgabeeinheit nachgeschaltet ist für eine fest eingestellte oder an einem am Gerätegehäuse (2) angebrachten Zeitvorgabeelement vorwählbare Befeldungszeit.

5. Befeldungsgerät nach Anspruch 4, **dadurch gekennzeichnet, dass** in Verbindung mit dem Wählschalter (6) die Befeldungszeit in der Stellung 10,0 Hz entsprechend einer "Arthrose-Therapie" auf 45 Minuten und in der Stellung 10,2 Hz entsprechend einer nichtinvasiven "Transkraniellen Stimulation" auf 60 Minuten fest eingestellt ist.

6. Befeldungsgerät nach einem der Ansprüche 1 bis 5, **dadurch gekennzeichnet, dass** die elektrische Leistung über ein Leistungs-Einstellelement am Gerätegehäuse (2) bis zu einem Maximalwert von 2mW kontinuierlich oder in Stufen einstellbar ist.

7. Befeldungsgerät nach einem der Ansprüche 1 bis 6, **dadurch gekennzeichnet, dass** die Energieversorgung der Elektronikeinheit einen geräteseitigen Akku mit zugeordneter Ladeeinheit und einen externen, mit einem Kabel verbindbaren Transformator mit einem Steckanschluss zu einer Gebäudesteckdose, insbesondere zum Anschluss an 230V, 50 Hz aufweist.

8. Befeldungsgerät nach einem der vorhergehenden Ansprüche, **dadurch gekennzeichnet, dass** die Halterung (19) zwei Bügelteile (20, 21) umfasst, die über eine einstellbare Schiebeführung zur Abstandseinstellung der Befeldungskapseln (12, 13) miteinander verbunden sind.

9. Befeldungsgerät nach einem der Ansprüche 1 bis 8, **dadurch gekennzeichnet, dass** eine Feldstärke-Begrenzungsvorrichtung für eine Begrenzung der von den Befeldungskapseln (12, 13) in die Umgebung abgestrahlten Hochfrequenz-Störpegel (HF-Störpegel) vorgesehen ist.

## Claims

1. Irradiation device for irradiating body parts of living beings with electromagnetic waves,
- comprising an electronics unit (3) with an energy supply (15), consisting of
- a radiofrequency oscillator stage (4) for producing a radiofrequency wave train at a frequency in the range of 130 MHz to 170 MHz,
- a low-frequency oscillator stage (5) which produces a frequency as modulation frequency,
- a modulation unit (7), which is supplied with the output of the radiofrequency oscillator stage (4) and the output of the low-frequency oscillator stage (5), for forming a modulated wave train, and
- a downstream output amplifier (8) as a power stage with a connected transmission antenna, in the emission region of which a body part (14) is positioned for irradiation purposes,
**characterized**
**in that** the transmission antenna is formed by two irradiation capsules (12, 13) connected at the output amplifier (8), said irradiation capsules each containing at least one magnetic coil and being placeable onto a body part (14), wherein the irradiation capsules (12,13) are connected to one another by way of a bracket-like or cap-like holder (19) in such a way that the active sides of said capsules face one another, wherein the holder (19) is placeable onto the body part (14) to be irradiated in such a way that the irradiation capsules (12, 13) rest opposite one another on the body part (14),
**in that** the electric power of the power stage (8) is limited to at most 2 mW,
**in that** the modulation frequency is set fixedly and unchangeably to 10.0 Hz and/or 10.2 Hz,
**in that** the radiofrequency oscillator stage (4) is set to a fixed and unchangeable frequency value in the range between 130 MHz and 170 MHz, and
**in that** the electronics unit (3) is contained in a device housing (2), on which an actuatable on/off switch (17) for the electronics unit (3) is applied for simple self-application and self-operation by a patient, and in that the holder (19) moreover forms the device housing (2) for receiving the electronics unit (3) with the on/off switch (17).

2. Irradiation device according to Claim 1, **characterized in that** the fixed and unchangeable radiofrequency value is set to 150 MHz.

3. Irradiation device according to Claim 1 or 2, **characterized in that** the modulation frequency is switchable between 10.0 Hz and 10.2 Hz by way of a selection switch (6) on the device housing (2) and the markings "osteoarthritis therapy" and "transcranial stimulation" are assigned to the two selection positions.

4. Irradiation device according to one of Claims 1 to 3, **characterized in that** a timing unit is disposed downstream of the on/off switch (17) for the purposes of a fixedly set irradiation time or an irradiation time which is preselectable at a timing element attached to the device housing (2).

5. Irradiation device according to Claim 4, **characterized in that**, in conjunction with the selection switch (6), the irradiation time is fixedly set to 45 minutes in the position 10.0 Hz corresponding to an "osteoarthritis therapy" and fixedly set to 60 minutes in the position 10.2 Hz corresponding to a non-invasive "transcranial stimulation".

6. Irradiation device according to one of Claims 1 to 5, **characterized in that** the electric power is adjustable continuously or in steps up to a maximum value of 2 mW by way of a power adjustment element at the device housing (2).

7. Irradiation device according to one of Claims 1 to 6, **characterized in that** the power supply of the electronics unit has a device-side accumulator with an assigned charging unit and an external, cable-connectable transformer with a plug-in connector to a building socket, in particular for connection to 230 V, 50 Hz.

8. Irradiation device according to one of the preceding claims, **characterized in that** the holder (19) comprises two bracket parts (20, 21) which are connected to one another by way of an adjustable sliding guide for the purposes of setting the spacing of the irradiation capsules (12, 13).

9. Irradiation device as claimed in one of Claims 1 to 8, **characterized in that** a field strength delimiting apparatus is provided for delimiting the radiofrequency noise level (RF noise level) emitted into the surroundings by the irradiation capsules (12, 13).

## Revendications

1. Appareil d'irradiation destiné à irradier des parties corporelles d'organismes vivants avec des ondes électromagnétiques,
- comportant une unité électronique (3) munie d'une alimentation en énergie (15), comprenant
- un étage oscillateur à haute fréquence (4) destiné à générer un train d'ondes à haute fréquence ayant une fréquence se situant dans la plage de 130 MHz à 170 MHz,
- un étage oscillateur à haute fréquence (5) qui génère une fréquence en tant que fréquence de modulation,
- une unité de modulation (7) à laquelle sont délivrées la sortie de l'étage oscillateur à haute fréquence (4) et la sortie de l'étage oscillateur à basse fréquence (5) pour former un train d'ondes modulé, et
- un amplificateur d'extrémité aval (8) en tant qu'étage de puissance muni d'une antenne d'émission raccordée, dans la zone de rayonnement de laquelle est positionnée une partie corporelle (14) à irradier,
**caractérisé en ce que** l'antenne d'émission est formée de deux capsules d'irradiation (12, 13) raccordées à l'amplificateur d'extrémité (8), qui contiennent respectivement au moins une bobine magnétique et qui peuvent être appliquées à une partie corporelle (14), dans lequel les capsules d'irradiation (12, 13) sont reliées l'une à l'autre de manière à ce que leurs faces actives soient dirigées l'une vers l'autre par l'intermédiaire d'une fixation (19) en forme d'étrier ou de capuchon, dans lequel la fixation (19) peut être placée sur la partie corporelle (14) à irradier de manière à ce que les capsules d'irradiation (12, 13) soient appliquées sur la partie corporelle (14) de manière opposée l'une à l'autre,
**en ce que** la puissance électrique de l'étage de puissance (8) est limitée à une valeur maximale de 2 mW,
**en ce que** la fréquence de modulation est réglée de manière fixe et non modifiable à 10,0 Hz et/ou à 10,2 Hz,
**en ce que** l'étage oscillateur à haute fréquence (4) est réglé à une valeur de fréquence fixe et non modifiable dans une plage comprise entre 130 MHz et 170 MHz, et
**en ce que** l'unité électronique (3) est contenue dans un boîtier d'appareil (2) sur lequel est monté un commutateur d'activation-désactivation actionnable (17) destiné à l'unité électronique (3) pour une application autonome et une utilisation autonome simple par un patient, et
**en ce que** la fixation (19) forme en outre le boîtier d'appareil (2) destiné à recevoir l'unité électronique (3) avec le commutateur d'activation-désactivation (17).

2. Appareil d'irradiation selon la revendication 1, **caractérisé en ce que** la valeur de la haute fréquence fixe et non modifiable est réglée à 150 MHz.

3. Appareil d'irradiation selon la revendication 1 ou 2, **caractérisé en ce que** la fréquence de modulation peut être amenée à basculer au moyen d'un commutateur de sélection (6) prévu sur le boîtier d'appareil (2) entre 10,0 Hz et 10,2 Hz et **en ce que** les indications "thérapie de l'arthrose" et "stimulation transcranienne" sont associées aux deux positions de sélection.

4. Appareil d'irradiation selon l'une quelconque des revendications 1 à 3, **caractérisé en ce qu'**une unité de saisie de temps est connectée en aval du commutateur d'activation/désactivation (17) pour un temps d'activation réglé de manière fixe ou pouvant être présélectionné sur un élément de saisie de temps monté sur le boîtier d'appareil (2).

5. Appareil d'irradiation selon la revendication 4, **caractérisé en ce qu'**en combinaison avec le commutateur de séléction (6) le temps d'irradiation est réglé de manière fixe à 45 minutes à la position 10,0 Hz correspondant à une "thérapie de l'arthrose" et à 60 minutes à la position 10,2 Hz correspondant à une "stimulation transcranienne" non invasive.

6. Appareil d'irradiation selon l'une quelconque des revendications 1 à 5, **caractérisé en ce que** la puissance électrique peut être réglée en continu ou par pas successifs par l'intermédiaire d'un élément de réglage de puissance sur le boîtier d'appareil (2) jusqu'à une valeur maximale de 2 mW.

7. Appareil d'irradiation selon l'une quelconque des revendications 1 à 6, **caractérisé en ce que** l'alimentation en énergie de l'unité électronique comporte un accumulateur côté appareil muni d'une unité de charge associée, et un transformateur pouvant être relié à un câble, comportant une borne enfichable dans une prise de courant d'un bâtiment, notamment pour un raccordement à 230 V, 50 Hz.

8. Appareil d'irradiation selon l'une quelconque des revendications précédentes, **caractérisé en ce que** la fixation (19) comprend deux parties d'étrier (20, 21) qui sont reliées l'une à l'autre par l'intermédiaire d'un coulisseau réglable pour le réglage de distance des capsules d'irradiation (12,13).

9. Appareil d'irradiation selon l'une quelconque des revendications 1 à 8, **caractérisé en ce qu'**un dispositif de limitation de l'intensité du champ est prévu pour limiter le niveau parasite à haute fréquence (niveau parasite HF) rayonné dans l'environnement par les capsules d'irradiation (12, 13).
